# EUROPEAN PATENT SPECIFICATION

(11) **EP 2 249 775 B1**
(45) Date of publication and mention of the grant of the patent: **12.04.2017**
(21) Application number: 09707501.4
(22) Date of filing: 06.02.2009
(51) Int. Cl.: A61K 8/35, A61K 8/39, A61K 8/42, A61K 31/205, A61Q 17/04, A61K 9/00

(54) **TOPICAL ANTIMICROBIAL COMPOSITIONS**
TOPISCHE ANTIMIKROBIELLE ZUBEREITUNG
COMPOSITIONS ANTIMICROBIENNES TOPIQUES

(30) Priority: 07.02.2008 GB 0802189
(43) Date of publication of application: 17.11.2010
(73) Proprietor: Reckitt Benckiser LLC, Parsippany, NJ 07054 (US)
(72) Inventor: BURT, Willard, Montvale, NJ 07645 (US); CLAYTON, James, Steven, Montvale, NJ 07645 (US); KOSTURKO, Richard, John, Montvale, NJ 07645 (US)
(74) Representative: Cawdell, Karen Teresa
(86) International application number: PCT/GB2009/000341
(87) International publication number: WO 2009/098476

(56) References cited:
- WO-A-00/15180
- WO-A-02/22083
- WO-A-2007/031300
- US-A- 5 955 062
- US-A1- 2003 125 224

## Description

The present invention generally relates to topical sanitizing compositions for application to the epidermis, e.g., hands, arms, legs, face, scalp as well as other body areas.

Topical compositions are well-known in the cosmetic, dermatological as well as in the pharmaceutical fields. Most topical compositions are intended to provide at least one, but generally provide multiple or more, specific benefits after being applied to the human skin. For example, personal care compositions which are primarily intended to be soaps for general cleaning of the human skin such as hand soaps or body wash soaps are well known in the fields of cosmetics and personal care products. While providing a primary cleaning benefit, such personal care compositions frequently also provide ancillary benefits such as moisturizing and nourishing the skin. Such personal care compositions which provide a good general cleaning benefit are usually based on one or more anionic soaps or anionic surfactants which are recognized to provide good cleaning and good foaming. However, such compositions typically provide only limited antimicrobial benefits.

WO0015180, WO2007031300, US2003125224 and WO0222083 describe examples of topical compositions providing antimicrobial benefits.

Also known to the art are topical compositions which are primarily directed to provide an antimicrobial benefit to the epidermis or other body part when applied thereto. Such typically take the form of lotions and are often largely comprised of an alcohol, usually ethanol with further constituents, e.g., thickeners. While often technically effective to provide an antimicrobial benefit, such compositions are also known to be prone to unduly dry the skin due to the evaporation of the alcohol which leaves the treated part of the epidermis with a chapped or dried feel. Further, such largely alcohol based topical compositions rarely provide a cleaning benefit. Additionally such largely alcohol based topical compositions may not have a high level of antimicrobial efficacy when applied to an epidermal surface for short contact times, such as may be modeled by test protocol and success requirements of the known-art protocols EN12054 (Chemical Disinfectants and antiseptics - Quantitative suspension test for the evaluation of antimicrobial activity), EN 1276 (Evaluation of Bactericidal Activity in Suspension - Dilution Neutralisation Method) and EN 1499 (Chemical Disinfectants and Antiseptics - Hygienic Handwash).
It is to these shortcomings in the art to which the current invention is directed.

In a first aspect of the present invention, there is described a topical antimicrobial composition for application to the epidermis which comprises:
- one or more quaternary ammonium compounds exhibiting germicidal efficacy; and
- is free of anionic soaps and anionic surfactants;
wherein:
the composition exhibits at least 3 log kill efficacy at 60 seconds contact time of at least two of the microorganisms selected from the group consisting of: S. *aureus, E. coli, P. aeruginosa* and *E.hirae*; and,
**characterised in that**
the composition further comprises a ternary thickening system comprising a benzophenone constituent, an oxyalkylenated constituent, and a fatty alkanolamide constituent.

An essential feature of these compositions is that they are are free of anionic surfactants and anionic soaps and are desirably also free of amphoteric surfactants. Yet, the compositions exhibit a satisfactory degree of foaming and exhibit excellent antimicrobial efficacy to treated surfaces, particularly a body surface, e.g., epidermis, skin, scalp and hair. The topical antimicrobial compositions are excellently suited for animal use, including human use.

The topical antimicrobial compositions exclude anionic surfactants and/or salt forms thereof. Examples of classes of anionic surfactants excluded are sulfates, sulfonates, phosphates, phosphonates and carbonates. Anionic soap surfactants are expressly excluded.

Preferably the topical antimicrobial compositions also exclude amphoteric surfactants. Examples of classes of amphoteric surfactants excluded are derivatives of aliphatic secondary and tertiary amines wherein at least one of the aliphatic substituents contains an anionic water-solubilizing group, e.g., a carboxy, sulfonate, or a sulfate group. Amphoteric surfactants which are especially desirably excluded are betaines.

### Cationic germicidal surfactants

A further essential feature of the surfactant constituent is the presence of one or more cationic surfactant constituents which provide an appreciable germicidal benefit. Such cationic surfactants are well known, and exemplary useful cationic surfactants may be one or more of those described for example in McCutcheon's Functional Materials(Vol. 2), North American Edition, 1998; Kirk-Othmer, Encyclopedia of Chemical Technology, 4th Ed., Vol. 23, pp. 481-541 (1997). These are also described in the respective product specifications and literature available from the suppliers of these cationic surfactants.

Especially preferred are alkyl ammonium halides which have the structural formula: 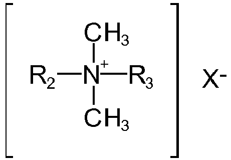 wherein R₂ and R₃ are the same or different C₈-C₁₂alkyl, or R₂ is C₁₂₋₁₆alkyl, C₈₋₁₈alkylethoxy, C₈₋₁₈alkylphenolethoxy and R₃ is benzyl, and X is a halide, for example chloride, bromide or iodide, or is a methosulfate anion. The alkyl groups recited in R₂ and R₃ may be straight-chained or branched, but are preferably substantially linear.

Particularly useful quaternary germicides include compositions which include a single quaternary compound, as well as mixtures of two or more different quaternary compounds. Such useful quaternary compounds are available under the BARDAC®, BARQUAT®, HYAMINE®, LONZABAC®, and ONYXIDE® trademarks.

Other cationic surfactants, although not specifically disclosed herein but known to the art may also be used. Preferred cationic surfactants are disclosed with reference to one of more of the following Examples. The cationic surfactants may be present as single compounds or as mixtures of two or more cationic surfactant compounds.

According to particularly preferred embodiments of the invention, one or more quaternary ammonium compounds which exhibit germicidal efficacy in the inventive compositions are necessarily present in amounts of from about 0.0001 - 5%wt., preferably in amounts of from about 0.001 - 2.5%wt., and more preferably in amounts of from 0.01 - 1%wt., based on the total weight of the composition of which it forms a part. Of quaternary ammonium compounds exhibiting germicidal efficacy and providing a germicidal benefit, those containing one or more benzene moieties are particularly preferred; especially preferred are benzalkonium halides (e.g., benzalkonium chlorides) and particularly preferred are benzethonium halides (e.g., benzethonium chlorides) as are demonstrated in one or more of the following examples.

### Non-ionic surfactants

The topical antimicrobial compositions optionally include a further nonionic surfactant constituent. Exemplary useful nonionic surfactants are those which include a hydrophobic base portion, such as a long chain alkyl group or an alkylated aryl group, and a hydrophilic chain portion comprising a sufficient number of ethoxy and/or propoxy moieties to render the nonionic surfactant at least partially soluble or dispersible in water.

Such nonionic surfactants include ethoxylated alkylphenols, ethoxylated and propoxylated fatty alcohols, polyethylene glycol ethers of methyl glucose, polyethylene glycol ethers of sorbitol, ethylene oxidepropylene oxide block copolymers, ethoxylated esters of fatty (C₆-C₂₄) acids, condensation products of ethylene oxide with long chain amines or amides, amine oxide compounds, and mixtures thereof.

The nonionic surfactants may be present as single compounds or as mixtures of two or more nonionic surfactant compounds.

The surfactant constituent may be present in any effective amount, and are preferably present in amounts of from 0.01 %wt. to 95%wt. based on the total weight of the composition of which they form a part. Preferably when the compositions are intended to be pourable compositions they comprise from 0.01 - 70%wt. surfactants. Preferably in such embodiments the surfactant constituent comprises (in order of increasing preference) at least 0.01%, 0.05%, 0.1%, 0.15%, 0.2%, 0.3%, 0.4%, 0.5%, 0.6%, 0.7%, 0.8%, 0.9%, 1%, 1.25%, 1.5%, 1.5% by weight, while concurrently the surfactant constituent comprises (in order of increasing preference) not in excess of 70%, 65%, 60%, 55%, 50%, 45%, 40%, 35%, 30%, 28%, 26%, 24%, 22%, 20, 18%, 17%, 16%, 15%, 14%, 13%, 12%, 11%, 10%, 9.5%, 9%, 8.5%, 8%, 7.5%, 7% by weight based on the total weight of the compositions of which they form a part.

### Ternary thickener

The present inventors have surprisingly found that many aqueous compositions may be thickened by combining a fatty alkanolamide constituent, an oxyalkylenated compound and a benzophenone compound or benzophenone derivative in order to provide a ternary thickening system which will boost the viscosity at least twice, preferably threefold and more preferably at least fourfold, especially preferably at least five-fold, yet more preferably at least eight-fold, and still more preferably at least ten-fold, even in the absence of conventional thickening constituents, e.g., clays, polyacrylate thickeners or polysaccharide based thickeners, as compared to aqueous compositions which exclude one of these three constituents. Such a comparison may be made by including the fatty alkanolamide, an oxyalkylenated compound in a composition, evaluating the viscosity and thereafter adding the benzophenone.

An essential feature of the composition of the present invention, therefore, is the presence of a ternary thickener system, comprising an oxyalkylenated constituent, a fatty alkanolamide constituent, and a benzophenone constituent.

### (1) Oxyalkylenated compounds

Exemplary oxyalkylenated compound(s) used in the composition of the invention comprise ethylene oxide groups (oxyethylenated compounds), propylene oxide groups (oxypropylenated compounds) or both (oxyethylenated/oxypropylenated compounds).

Suitable oxyalkylenated compounds include, in particular, polyethylene glycols, polyethylene glycol esters and/or polypropylene glycol esters, polyethylene glycol ethers and/or polypropylene glycol ethers, alkoxylated aryl derivatives and in particular ethoxylated aryl polyol derivatives, oxyalkylenated and in particular oxyethylenated triesters of glycerol and of fatty acids, ethoxyethylenated urethane derivatives modified with alkyl chains, and mixtures thereof.

Exemplary polyethylene glycols which may be used in the composition of the invention include ethylene oxide polycondensates having a number of ethylene oxide (EO) units from 10 to 50 000 and preferably from 14 to 10 000.

Useful polyethylene and/or polypropylene glycol esters have the formula:

RCOO-(EO)ₘ-(PO)ₙ-R'

in which m has a value of 0 to 300, n has a value of 0 to 300 the sum of m and n is 6 or greater, and R and R' represent, independently of each other, hydrogen or a saturated or unsaturated, linear or branched, hydroxylated or non-hydroxylated alkyl chain containing from 1 to 30 carbon atoms and preferably from 12 to 22 carbon atoms, or an aryl chain, with the proviso that R and R' are not simultaneously hydrogen.

Useful polyethylene and/or polypropylene glycol ethers are compounds of formula:

R-(EO)ₘ-(PO)ₙ-R'

in which m has a value of 0 to 300, n has a value of 0 to 300 the sum of m and n is 6 or greater, and R and R' represent, independently of each other, hydrogen or a saturated or unsaturated, linear or branched, hydroxylated or non-hydroxylated alkyl chain containing from 1 to 30 carbon atoms and preferably from 12 to 22 carbon atoms, or an aryl chain, with the proviso that
R and R' are not simultaneously hydrogen.

Further suitable oxyalkylenated compounds include oxyethylenated derivatives of fatty acid esters or of fatty alcohol ethers and of a polyol such as glycerol, sorbitol, glucose or pentaerythritol. Further suitable compounds include oxyalkylenated glyceryl triesters of fatty acids.

Preferred oxyalkylenated compound of the topical compositions are polyethylene glycol diesters such as polyethylene glycol distearates ethoxylated with 50 - 350 units ethylene oxide. A preferred material is Hallstar PEG 6000 DS, described to be a polyethylene glycol distearate ethoxylated with 150 units of ethylene oxide.

Other oxyalkylenated compounds falling within the above descriptions, although not specifically disclosed herein but known to the art may also be used. Preferred oxyalkylenated compounds are disclosed with reference to one of more of the following Examples. The oxyalkylenated compounds may be present as single compounds or as mixtures of two or more oxyalkylenated compounds.

The oxyalkylenated compound(s) may be present in any effective amount, and are preferably present in amounts of from 0.01 %wt. to 25%wt. based on the total weight of the composition of which they form a part. Preferably however the oxyalkylenated compounds comprises 0.1 - 15%wt., preferably from 0.5 - 10%wt. based on the total weight of the topical antimicrobial compositions of which they form a part.

### (2) Fatty alkanolamide

A second constituent of the ternary thickening system of the invention is a fatty alkanolamide, examples of which include but are not limited to: cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA, and mixtures thereof. Two or more fatty alkanolamides may be used to form the fatty alkanolamide constituent of the invention.

The fatty alkanolamide constituent may be present in any useful amount, but advantageously is present in an amount of from 0.001 - 95%wt. based on the total weight of the composition of which it forms a part. Advantageously when the compositions are intended to be pourable compositions they comprise from 0.01 - 70%wt. of the alkanolamide constituent. Preferably in such embodiments the alkanolamide constituent comprises (in order of increasing preference) at least 0.01%, 0.05%, 0.1%, 0.15%, 0.2%, 0.3%, 0.4%, 0.5%, 0.6%, 0.7%, 0.8%, 0.9%, 1%, 1.25%, 1.5%, 1.5% by weight, while concurrently the alkanolamide constituent comprises (in order of increasing preference) not in excess of 70%, 65%, 60%, 55%, 50%, 45%, 40%, 35%, 30%, 28%, 26%, 24%, 22%, 20, 18%, 17%, 16%, 15%, 14%, 13%, 12%, 11%, 10%, 9.5%, 9%, 8.5%, 8%, 7.5%, 7% by weight based on the total weight of the compositions of which they form a part.

The fatty alkanolamide constituent is an essential constituent of the present invention.

In addition to a thickening benefit, the fatty alkanolamide constituent may provide an ancillary foam boosting benefit as well.

### (3) Benzophenone

The third constituent of the ternary thickening system of the invention is a benzophenone constituent which comprises one or more benzophenone compounds and/or derivatives thereof. Examples of such compounds include those which may be represented by the following formula 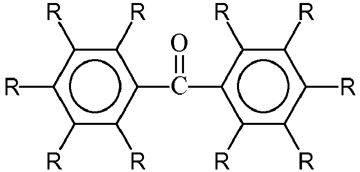 wherein each "R" independently represents a hydrogen atom, a halogen atom, a hydroxyl group, a nitro group, a carboxyl group, a lower alkoxycarbonyl group, an alkyl group, an alkenyl group, a lower alkoxy group, a formyl group, an acyl group or an --N(R')(R") group, wherein R' and R" each represents a hydrogen atom, a lower alkyl group, a lower alkenyl group, a phenyl group, a benzyl group or an acyl group. Acids and salts of the foregoing compounds are also contemplated as being useful as well. In the foregoing, examples of acyl groups include acetyl groups; the carbon number of an alkyl group and an alkenyl group is preferably from 1 to 18; and the term "lower" preferably means that from 1 to 4 carbons are present. Preferred benzophenone compounds and/or derivatives thereof include 2-hydroxy-4-methoxy benzophenone, 2-hydroxy-4-methoxy-4'-methyl benzophenone, n-hexyl 2-(4-diethylamino-2-hydroxybenzoyl)benzoate, 2,2'-dihydroxy-4-methoxy benzophenone as well as sulphonic acid derivatives of benzophenones, e.g., 2-hydroxy-4-methoxybenzophenon-5-sulphonic acid. Certain of these materials are currently marketed under the UVINUL tradename by BASF.

The benzophenone constituent is present in amounts of up to about 20%wt., but are preferably included in amounts of from about 0.1 - 5%wt. based on the total weight of the composition of which they form a part. The benzophenone constituent is an essential constituent of the present invention.

Preferably the ternary thickening system comprises from 0.01 to 25 wt% of the topical antimicrobial composition, more preferably from 0.1 to 20wt%, further preferably from 0.5 to 10 wt%, especially from 1 to 5 wt%.

Especially preferred respective weight ratios of the respective fatty alkanolamide, an oxyalkylenated compound and a benzophenone compounds or benzophenone derivative are disclosed with reference to one or more of the examples

It is to be understood that the ternary thickening system viz., the fatty alkanolamide, an oxyalkylenated compound and a benzophenone compound or benzophenone derivative, is preferably present in the topical antimicrobial compositions described herein in the absence of clays, polyacrylate thickeners or polysaccharide based thickeners.

### pH adjusters/buffers

The topical antimicrobial compositions, as well as non-topical compositions which include the ternary thickening system of constituents disclosed herein may be of any pH in the range of 0 - 14, but preferred topical antimicrobial compositions are acidic in nature and the inventors have observed that good antimicrobial efficacy is provided when the inventive foaming topical compositions are also maintained in a specific acidic pH range, that is, pH of about 7 or less, preferably about 6 or less, especially 5.5 or less.

Particularly preferred pH values are those in the range of from 4.0 - 7.0, more preferably 4.0 - 6.0, further preferably from 4.5 - 5.5, and especially preferably between 5 - 5.5. Further preferred pH values are disclosed with reference to one or more of the Example compositions.

The pH of the compositions may be established or adjusted by the use of suitable amounts of an acid, e.g., one or more inorganic acids, mineral acids, but preferably by the use of one or more organic acids. Generally useful organic acids are those which contain from 1 to about 6 carbon atoms and include at least one carboxyl group (--COOH) in its structure. Exemplary useful organic acids include: linear aliphatic acids; dicarboxylic acids; acidic amino acids; and hydroxy acids as well as acid salts of these organic acids.

Preferred useful organic acids include citric acid, cresylic acid, dodecylbenzene sulfonic acid, phosphoric acid, salicylic acid, sorbic acid, sulfamic acid, acetic acid, benzoic acid, boric acid, capric acid, caproic acid, cyanuric acid, dihydroacetic acid, dimethylsulfamic acid, polyacrylic acid, 2-ethyl-hexanoic acid, fumaric acid, I-glutamic acid, isopropyl sulfamic acid, naphthenic acid, oxalic acid, phosphorous acid, valeric acid, benzene sulfonic acid, xylene sulfonic acid, sulfonic acids, maleic acid, acetic acid, adipic acid, formic acid, lactic acid, butyric acid, gluconic acid, malic acid, tartaric acid, as well as glycolic acid. In certain preferred embodiments of the invention the sole acids present are one or more of: citric acid, and/or lactic acid to the exclusion of other acids.

These acids can be used singly or as a mixture of two or more individual acids. While they may be present in any effective amount in order to attain a desired acidic pH, advantageously they are present in an amount of from about 0 - 15%wt., more preferably from 0.001 - 10%wt., yet more preferably from 0.01 - 5%wt. based on the total weight of the compositions of which they form a part.

It is to be expressly understood that topical antimicrobial compositions may be formed without the necessity to add one or more acids, dependant upon the nature of the other constituents which may be present.

In order to further adjust the pH of the inventive compositions, one or more pH adjusting agents and/or pH buffers may be included in the topical antimicrobial compositions in effective amounts. When present, the pH adjusting agent, especially the pH buffers are present in an amount effective in order to maintain the pH of the inventive composition within a desired or a target pH range. Advantageously they may be included in generally minor amounts such as from 0.001 - 1.5 %wt. but desirably are present in amounts from 0.01 - 1%wt. Exemplary and preferred pH buffers and pH adjusting agents are described with reference to one or more of the following Examples.

### Organic solvents

The compositions of the invention may include one or more organic solvents, such as alcohols, glycols, acetates, ether acetates and glycol ethers. Exemplary alcohols include C₃-C₈ alcohols which may be straight chained or branched, and which are specifically intended to include both primary and secondary alcohols. Exemplary glycol ethers include those glycol ethers having the general structure Rₐ-O-R_{b}-OH, wherein Rₐ is an alkoxy of 1 to 20 carbon atoms, or aryloxy of at least 6 carbon atoms, and R_{b} is an ether condensate of propylene glycol and/or ethylene glycol having from one to ten glycol monomer units. Preferred organic solvents include C₁-C₄ alkylene glycols, e.g. propylene glycol which is also known to be a humectant, and C₁-C₄ monohydric alcohols, e.g, ethanol which may provide an increase in antimicrobial efficacy. When present the organic solvent constituent comprises from about 0.001 - 15%wt., more preferably from 0. 1 - 10%wt., yet more preferably from 0.5 - 5%wt. based on the total weight of the compositions of which they form a part. In certain preferred embodiments, the organic solvent constituent is necessarily present, while in other certain preferred embodiments the organic solvent constituent is necessarily absent.

### Log kill/effectiveness

The topical antimicrobial compositions provided according to the present invention exhibit at least 3 log₁₀ kill efficacy, preferably at least 4 log₁₀ kill efficacy and especially preferably at least 5 log₁₀ kill efficacy at 60 seconds contact time of at least two, preferably at least three and most preferably at least four of microorganisms selected from the group consisting of: S. *aureus, E. coli, P. aeruginosa* and *E.hirae,* according to the protocols of prEN 12054. As is recognized in the art, this test "prEN12054 - Chemical Disinfection and Antiseptics - Products for Hygienic and Surgical Handrub and Handwash, Bactericidal Activity - Test Methods and Requirements (Phase 2, Step 1)" is one which is particularly difficult to pass with a plurality of the designated test organisms at short contact times. Thus the preferred embodiments of the topical antimicrobial compositions taught herein provide a significant advance over prior art compositions which fail to exhibit at least 3, preferably 4 and particularly preferably 5 log₁₀ kill efficacy, at 60 seconds contact time of at least three, preferably all four of microorganisms selected from the group consisting of: S. *aureus, E. coli, P. aeruginosa* and *E.hirae* according to prEN12054. Most preferred topical antimicrobial compositions are those which provide at least a 4 log₁₀ kill, preferably at least a 5 log₁₀ kill efficacy at 60 seconds against *P. aeruginosa* according to one or more of the known-art protocols EN12054 (Chemical Disinfectants and antiseptics - Quantitative suspension test for the evaluation of antimicrobial activity), EN1276 (Evaluation of Bactericidal Activity in Suspension - Dilution Neutralisation Method) and EN 1499 (Chemical Disinfectants and Antiseptics - Hygienic Handwash).

### Container

While topical antimicrobial compositions which are readily pourable are preferred, it is to be understood that the topical antimicrobial compositions may be provided in compositions which are essentially water-thin, and have a viscosity as low as 1cP, or as an essentially solid form, or compositions which are stiff but spreadable, or pasty. Thus, the viscosity of the topical antimicrobial compositions is expected to be extremely broad.

The topical antimicrobial compositions may be characterized in having a viscosity of at least 500 cP at 25°C, preferably between 1000 - 50,000 cP, more preferably between about 1000 - 25,000 cP, and especially preferably between 1500 - 20,000 cP at 25°C. The viscosity may be determined using conventional quantitative methods, e.g., via the use of a Brookfield Type LV3 viscometer rotating at 12 rpm at 25°C.

The amount of water present may thus vary widely and depends in great part on the selected constituents and the desired product form. Advantageously, water is added to order to provide to 100% by weight of the compositions of the invention. When present, water may be present in widely ranging amounts, up to about 98%wt. of the total weight of the composition of which it forms a part, although it is frequently present in reduced amounts, e.g., 95%, 92.5%, 90, 85%, 80%, 75%, 70%, 65%, 60%, 55%, 50%, 45%, 40%, 35%, as well as even lesser amounts of 30%, 25%, 20%, 15%, 10%, 5%wt. based on the product form and further based on the total weight of the composition of which it forms a part. Compositions of the invention in which no water is added to the constituents "as supplied" from their respective suppliers are also contemplated, as frequently one or more constituents ma y be supplied with an aqueous or aqueous/organic liquid carrier.

The composition can be packaged in a suitable container to suit its viscosity and intended use by the consumer. For example, a lotion or cream can be packaged in a bottle, or can be packaged with a propellant in a propellant-driven aerosol device or a may be packaged in a container fitted with a manually operable pump. When the compositions of the invention have higher viscosities and is in the form of a paste, gel or cream it may conveniently be provided in a resealable container with a relatively wide opening, e.g., a jar, tin, tub or bottle with a removable and replaceable cap or cover. Forms of the composition which have low viscosities may be provided in bottles or flasks from which they be dispensed by pouring, or by pumping such as via a manually pumpable trigger pump or manually operable trigger spray pump. The inventive composition can be provided and stored in a non-deformable bottle but more preferably is provided in a squeezable container, such as a tube or deformable bottle which provides for easy dispensing of the composition by the consumer. Thus a further aspect of the invention provides a closed container containing the inventive composition as described herein. When the composition is in a solid form such as a bar, tablet or cake, this may be packaged in a wrapper which is torn away and discarded, or provided in a tray or other container in which the unused portion of the solid form may be stored between uses by a consumer.

### Further constituents/components

The topical antimicrobial compositions of the invention may include one or more further optional constituents which may be used to impart one or more desired esthetic or technical benefits to the said compositions. In certain preferred embodiments of the invention, one or more of the following recited optional constituents may be considered as essential constituents according to a particular preferred embodiment. Such optional constituents include additives and adjuvants which are conventional in the cosmetic, pharmaceutical or dermatological field, such as hydrophilic or lipophilic gelling agents, hydrophilic or lipophilic active agents, further thickeners, humectants, opacifiers, preservatives, antioxidants, solvents especially organic solvents, pH adjusting agents, pH buffers, chealating agents, fragrances, fragrances or other materials which provide an aromatherapy benefit, fillers, preservatives, dyestuffs or colorants, and light stabilizers including UV absorbers. The total amounts of these various additives and adjuvants are those conventionally used in the field, and, for example, range from 0.01% to 75%, preferably 0.01% - 50%wt. of the total weight of the composition.

The inventive compositions may optionally include further thickeners which are not found to deleteriously affect the favorable technical characteristics of the present invention, especially the antimicrobial characteristics. Coming into consideration are one or more of polysaccharide polymers selected from cellulose, alkyl celluloses, alkoxy celluloses, hydroxy alkyl celluloses, alkyl hydroxy alkyl celluloses, carboxy alkyl celluloses, carboxy alkyl hydroxy alkyl celluloses, naturally occurring polysaccharide polymers such as xanthan gum, guar gum, locust bean gum, tragacanth gum, or derivatives thereof, polycarboxylate polymers, polyacrylamides, polyacrylate cross-polymer thickeners, clays, and mixtures thereof.

The topical antimicrobial compositions may include a cosmetic particulate, which may be any particulate material which is a solid at room temperature, which does not deleteriously react chemically with balance of the constituents of the inventive composition. Advantageously the cosmetic particulate is insoluble in balance of the constituents of the topical antimicrobial compositions.

Exemplary materials useful for the cosmetic particulate constituent include inorganic particulates, polymeric organic particulates, carbonates, hollow silica microspheres, glass microcapsules, ceramic microcapsules, inorganic pigments, crystalline and microcrystalline waxes derived from plants, mineral oils or petroleum, hollow polymer microspheres, starches, alginates, organic dyestuffs or pigments, and mixtures thereof. Mixtures of two or more cosmetic particles may be used to provide the cosmetic particulate constituent. Preferred as the cosmetic particulate constituent are materials which provide an exfoliating benefit.

A preferred class of cosmetic particulate materials are based on synthetically occurring or synthetic waxes inclusive of microcrystalline waxes. If included in the compositions of the invention, the cosmetic particulate constituent of the invention may be provided in any effective amount, advantageously from at least 0.01 %wt., preferably at least 0.05%wt, and most preferably at least 0.1 %wt of the topical antimicrobial composition. Similarly advantageously the cosmetic particulate constituent is present in not more than 10%wt., preferably not more than 5%wt, and yet more preferably not more than 2%wt, and most preferably not more than 2%wt of the topical antimicrobial composition of which it forms a part.

The topical antimicrobial compositions may include one or more fillers in the form of powders. By way of non-limiting examples these powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof. When present in a composition, the one or more fillers may be present in amounts of up to about 5%wt., preferably are present in amounts of from about 0.001 %wt. to about 5%wt. based on the total weight of the topical composition of which it forms a part.

The topical compositions may also a cationic polyquaternium-type polymer. Such materials are well known to the art of topical compositions and are described in the literature, particularly in the International Cosmetic Ingredient Dictionary and Handbook, Volume 2 (9th Edition, 2002), at pages 1311 - 1319. Other polyquaternium compounds although not specifically elucidated here may also be utilized in the present inventive compositions.

When present in the topical antimicrobial compositions, the one or more cationic polyquaternium-type polymers are advantageously present in amounts of from about from 0.001 - 5 %wt., preferably in amounts from 0.01 - 2%wt., but are most desirably present in reduced weight percentages from about 0.05 - 1%wt. based on the total weight of the topical antimicrobial composition of which they form a part.

One optional constituent which may be included in the inventive compositions is a latex. Such are used to provide opacification of the composition, and are also referred to in the art as "opacifiers". Such are materials which are typically emulsions, dispersions or suspensions of a a water insoluble polymer or copolymer in an carrier. The carrier may be aqueous, an aqeueous/organic solvent mixture or organic solvent. The latex may be based on a homopolymer, or on copolymer formed from styrene, alpha-methylstyrene, divinylbenzene, acrylic acid or C₁ -C₂₀ esters thereof, methacrylic acid or C₁ -C₂₀ esters thereof, (meth)acrylamide, maleic acid, vinyl acetate, crotonic acid, vinyl neodecanoate and butenoic acid.

Particularly preferred latexes useful in the present invention are latexes presently commercially available under the trademark ACUSOL (ex. Rohm & Haas Inc.). When present in a composition, the latex may be present in amounts of up to about 5%wt., preferably are present in amounts of from about 0.001 %wt. to about 3%wt., preferably are present in amount from about 0.1 %wt. to about 1.2%wt, and most preferably are present in amounts of from about 0.1 %wt. to about 1%wt., based on the total weight of the topical composition of which it forms a part. Concurrently the amount of the of the water-insoluble polymer present in the latex may range from about 0.01 to about 90%, preferably from about 0.1 to about 60%, optimally from about 10 to about 50% by weight of the latex.

The topical antimicrobial compositions may include one or more preservatives. Most preferred preservatives are those commercially available preservative comprising a mixture of 5-chloro-2-methyl-4-isothiazolin-3-one and 2-methyl-4-isothiazolin-3-one marketed under the trademark KATHON CG/ICP as a preservative composition (ex. Rohm and Haas Inc.). Further useful preservative compositions include KATHON CG/ICP II (ex. Rohm and Haas Inc.), PROXEL (ex. Zeneca), SUTTOCIDE A (ex. Sutton Laboratories) as well as TEXTAMER 38AD (ex. Calgon Corp.) When present the preservative is included in any amount found to be effective in retarding or inhibiting the grown of undesired microorganisms in the topical antimicrobial compositions, particularly during storage for several months at room temperature. When present in a composition, in accordance with certain of the preferred embodiments, the preservative composition is advantageously present in amounts of up to about 1.5%wt., preferably are present in amounts of from about 0.00001 %wt. to about 0.5%wt., and most preferably is present in an amount of from about 0.0001 %wt. to 0.25%wt. based on the total weight of the topical composition of which it forms a part.

The topical antimicrobial compositions may include a fragrance constituent, which may be based on natural and synthetic fragrances and most commonly are mixtures or blends of a plurality of such fragrances, optionally in conjunction with a carrier such as an organic solvent or a mixture of organic solvents in which the fragrances are dissolved, suspended or dispersed. When present in a composition, in accordance with certain of the preferred embodiments, the fragrance constituent may be present in any effective amount such that it can be discerned by a consumer of the topical composition, however is advantageously present in amounts of up to about 1%wt., preferably are present in amounts of from about 0.00001 %wt. to about 0.5%wt., and most preferably is present in an amount of from about 0.0001 %wt. to 0.25%wt. based on the total weight of the composition of which it forms a part.

The inventive topical antimicrobial compositions may include one or more colorants, e.g, dyes or pigments which are known to the art be useful in cosmetic or topical compositions which may be used to impart a desired color or tint to the inventive compositions. Any colorant which is compatible with the other constituents forming the topical compositions may be used and such may be present in any amount effective to achieved the desired visual effect. Advantageously one or more colorants may be added in amounts of about 0.001 %wt. to about 0.1% by weight, based on the total weight of the composition of which the colorant(s) forms a part.

The topical antimicrobial compositions may comprise one or more humectants, which may be used singly or two or more humectants may be included in topical antimicrobial compositions of the invention. Of the humectants, aloe vera in one or more of its forms is preferred as being a naturally derived product. When present, in accordance with certain of the preferred embodiments, one or more humectants may be included in effective amounts, advantageously from 0.01 - 2.5%wt., preferably from 0.01 - 2%wt. based on the total weight of the composition of which it forms a part.

The topical antimicrobial compositions of the invention may include one or more essential oils which are selected to provide a so-called "aromatherapy benefit" to the user. Essential oils are complex mixtures of different organic molecules, such as terpenes, alcohols, esters, aldehydes, ketones and phenols. Such essential oils are frequently extracted from naturally occurring botanical sources such as flowers, stems, leaves, roots and barks of aromatic plants. While essential oils may be used singly, it is also common to utilize blends of essential oils in order to provide a conjunctive aroma benefit, and possibly a therapeutic benefit as well.

As used in the present invention, these one or more essential oils providing an aromatherapy benefit are present in an amount about 0.00001 wt. % to about 1 wt. %, based on the total weight of the composition. Preferably, the one or more essential oils providing an aromatherapy benefit are present in an amount about 0.00005 wt. % to about 0.75 wt. %, and more preferably about 0.0001 wt. % to about 0.5 wt. % of the total weight of the composition. It is to be understood that these one or more essential oils providing an aromatherapy benefit may be used with our without the optional fragrancing constituent recited previously and may be used wholly or partially in place of said fragrancing constituent.

The topical antimicrobial compositions may include one or more antioxidant constituents. Examples of antioxidants include but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, glutathione, and ascorbic acid and ascorbic acid derivatives (e.g., ascorbyl palmitate and ascorbyl polypeptide). Oil-soluble antioxidants suitable for use in the topical antimicrobial compositions of this invention include, but are not limited to, butylated hydroxytoluene, retinoids, tocopherols e.g., tocopherol acetate, tocotrienols, and ubiquinone. Natural extracts containing antioxidants suitable for use in the topical compositions of this invention, include but not limited to, extracts containing flavonoids and isoflavonoids and their derivatives, extracts containing resveratrol and the like. Examples of such natural extracts include grape seed, green tea, pine bark, propolis, and the like. When present the total amount of such antioxidants are usually not in excess of 5%wt, preferably are present in amounts of from 0.0001 - 4%wt. based on the total weight of the topical antimicrobial compositions of which it forms a part. In certain preferred embodiments an one or more antioxidants constituents are necessarily present.

Optionally the topical antimicrobial compositions may include one or more vitamins. When present, in accordance with certain of the preferred embodiments, one or more vitamins may be included in effective amounts, advantageously from 0.0001 - 1%wt., preferably from 0.001 - 0.75%wt. based on the total weight of the topical antimicrobial compositions of which it forms a part.

The topical antimicrobial compositions may include one or more light stabilizers as well as UV absorbers. Such materials are known to be useful in cosmetic or topical compositions and impart a degree of stability to the compositions which may comprise one or more components which may be deleteriously affected when exposed to certain sources of light, e.g., sunlight, fluorescent light sources. Other such materials are known to stabilize or improve the effect of colorants which may be present in the compositions. Any cosmetically acceptable material or compound which provides protection for one or more of the constituents in the inventive compositions from photolytic degradation or photo-oxidative degradation may be used.

Exemplary and preferred such materials which are presently commercially available include one or more of: CIBAFAST H liquid, described to be sodium benzotriazolyl butylphenol sulfonate with Buteth-3 and tributyl citrate; TINOGARD HS described to be sodium benzotriazolyl butylphenol sulfonates; TINOGARD AS described to be bumetrizole; TINOGARD TL described to be benzotriazolyl dodecyl p-cresol; and TINOGARD Q described to be tris(tetramethyl-hydroxypiperidinol) citrate, all of which are presently commercially available from Ciba Specialty Chemicals (Muttenz, CH.).

When present, the one or more light stabilizers as well as UV absorbers may be included in any effective amount; advantageously such materials are present in amounts of from 0.0001 - 1%wt., preferably from 0.001 - 0.5%wt. based on the total weight of the topical antimicrobial composition of which it forms a part.

The inventive topical antimicrobial compositions may include one or more chelating agents. Preferred chelating agents include acids and salts, especially the sodium and potassium salts of ethylenediaminetetraacetic acid (EDTA), diethylenetriaminepentaacetic acid, N-hydroxyethylethylenediaminetriacetic acid, and of which the sodium salts of ethylenediaminetetraacetic acid may be particularly advantageously used. Such chelating agents may be omitted, or they may be included in generally minor amounts such as from 0.001 - 0.5 %wt. based on the weight of the chelating agents and/or salt forms thereof. Desirably, such chelating agents are included in the present inventive composition in amounts from 0.01 - 0.5%wt., but are most desirably present in reduced weight percentages from about 0.01 - 0.2%wt.

A particularly preferred embodiment of the present invention comprises:
- from 0.001 to 2.5 wt% of one or more quaternary ammonium compounds exhibiting germicidal efficacy;
- from 0.01 to 25 wt% of a ternary thickener system comprising a benzophenone constituent, an oxyalkylenated constituent, and a fatty alkanolamide constituent; and,
- from 0.01 to 7 wt% of a nonionic surfactant.

An especially preferred embodiment of the present invention comprises:
- from 0.01 to 1 wt% of one or more quaternary ammonium compounds exhibiting germicidal efficacy;
- from 0.1 to 10 wt% of a ternary thickener system comprising a benzophenone constituent, an oxyalkylenated constituent, and a fatty alkanolamide constituent;
- from 0.01 to 1 wt% of a nonionic surfactant;
- from 0.5 to 5 wt% of an organic solvent; and,
- an organic carboxylic acid.

In a further aspect, the present invention also contemplates a method for providing a cleaning and/or providing an antimicrobial benefit to skin or other topical surface which method contemplates the topical application of the aqueous topical antimicrobial compositions as described herein in a cleaning and/or antimicrobially effective amount. Preferably according to the foregoing method, a antimicrobial benefit is provided to the skin or other topical surface to which the composition has been applied.

While the topical antimicrobial compositions disclosed herein find a primary use in application to the skin to provide a cleaning and/or antimicrobial benefit thereto and is contemplated as being provided in a dispenser for use in such a treatment, it is to be understood that this is not to be understood as a limiting definition and that other forms and other uses of the present inventive composition, such as face lotion, milky lotion, cream, face cleansing cream, massage materials, liquid toilet soap, as well as in hair care products such as shampoo, rinse or other hair or scalp treatment are expressly contemplated as being within the scope of the present invention. The topical antimicrobial compositions of the invention can be formulated as a lotion, a cream or a gel, which may be transparent, translucent or opaque. In certain preferred embodiments the topical antimicrobial compositions is provided as a translucent or transparent, preferably a transparent composition.

It is to be further expressly understood that topical application of the topical antimicrobial compositions disclosed herein may be applied to the skin on any part of the body, including the skin on the face, neck, chest, back, arms, axilla, hands, legs, and scalp. The topical antimicrobial compositions disclosed herein may also be used on the hair.

It is contemplated that in use, the consumer dispenses a quantity of the topical antimicrobial composition described herein and applied it to the skin or any other part of the body which has preferably been wetted with water (e.g, rinsed) prior to application of the said composition. The topical antimicrobial compositions may be rubbed into the applied skin or other part of the body by the consumer to generate a lather or foam, and thereafter it is expected that the treated area is rinsed by the consumer under a stream of running water, e.g, in a shower or by immersion into water, e.g, a bath. Thereafter the skin or other parts of the body of the consumer is permitted to air dry or the use of one or more towels to absorb excess moisture is also contemplated. Thus, a further aspect of the invention is directed to the use of the topical antimicrobial compositions as described herein.

The following examples below illustrate exemplary formulations as well as preferred embodiments of the invention.

### Examples

A number of topical antimicrobial compositions according to the present invention were produced according to the invention described below were produced in by mixing measured amounts of the identified constituents in water until a homogenous mixture was formed. Thereafter the final constituent the benzophenone was added during stirring at which time it was observed that the viscosity of the composition increased significantly.

In the following compositions, the constituents were used "as supplied" from their respective suppliers and may constitute less than 100%wt. "actives", or may have been supplied as constituting 100%wt "active" of the named compound, as indicated in the following Tables 1, 2 and 3. The identity of the individual constituents used are described more fully in Table 3.

To each of the compositions was included deionized water in "quantum sufficient" (q.s.) in order to provide 100 parts by weight of the specific composition.

### Antimicrobial Efficacy:

The topical antimicrobial efficacy of a composition according to the invention was evaluated in accordance with prEN 12054 - Chemical Disinfectants and antiseptics - Quantitative suspension test for the evaluation of antimicrobial activity. This test is used to evaluate the antimicrobial efficacy of test compositions for hygienic and surgical handrub and handwash applications. As is recognized in the art, the requirements of prEN 12054 are particularly difficult to satisfy particularly at short contact times of the test.

The compositions of Table 1 were evaluated in accordance with prEN 12054, for contact times of 60 seconds. For each challenge microorganism, 2 replicates were used in each test. Table 2 reports the log₁₀ reduction achieved for the identified compositions. As is evident from the above, particularly preferred were compositions which exhibited at least 4 log₁₀ reduction, yet more preferably at least 5 log₁₀ reduction of each of S. *aureus, E. coli, P. aeruginosa* and *E.hirae,* according to the protocols of prEN12054.

Also indicated in Table 2 are the viscosity of the compositions, which were measured at 20°C and 25°C using a Brookfield Type LV3 viscometer, using the recommended spindle and rotating at 12 rpm.

As is evident from the reported results the compositions exhibited excellent antimicrobial activity according to the protocols of prEN12054.

Additionally while not individually reported herein, preferred compositions exhibited excellent storage stability when stored at elevated temperatures of 40°C for intervals of 2 to 4 weeks.

**Table 1**

| | Ex.1 | Ex.2 | Ex.3 | Ex.4 | Ex.5 | Ex.6 | Ex.7 | Ex.8 | Ex.9 | Ex.10 | Ex.11 |
|---|---|---|---|---|---|---|---|---|---|---|---|
| polyethylene glycol distearate | 1.9 | 1.75 | 1.75 | 1.75 | 1.83 | 1.83 | 1.83 | 2.0 | 2.0 | 1.4 | 1.83 |
| lauramine oxide (30%) | 2.75 | 2.75 | 2.75 | 2.75 | 2.75 | 2.75 | 2.75 | 2.5 | 2.5 | 3.5 | 2.5 |
| lauramide DEA | 1.0 | 1.0 | 1.0 | 1.0 | 1.0 | 1.0 | 1.0 | 1.0 | 1.0 | 0.7 | 1.0 |
| bis-[polyoxypropylene (2), polyoxyethylene (10) myristyl ether | 0.1 | 0.1 | 0.1 | 0.1 | 0.1 | 0.1 | 0.1 | 0.1 | 0.1 | 0.1 | 0.1 |
| cetyl trimethyl ammonium chloride (30%) | 5.8 | 5.8 | 5.8 | 5.8 | 5.8 | 5.8 | 5.8 | 5.8 | 5.8 | 5.0 | 5.8 |
| benzalkonium chloride (50%) | 0.26 | 0.26 | 0.26 | 0.26 | 0.26 | 0.18 | 0.26 | 0.32 | 0.26 | -- | -- |
| benzethonium chloride (100%) | -- | -- | -- | -- | -- | -- | -- | -- | -- | 0.13 | 0.13 |
| cocoamidopropyl betaine (30%) | -- | -- | -- | -- | -- | -- | 3.0 | 2.5 | -- | -- | -- |
| glycerine | 4 | 4 | 4 | 4 | 4 | 4 | 2 | 4 | 4 | 2 | 2 |
| propylene glycol | 0.1 | 0.1 | 0.1 | 0.1 | 0.1 | 0.1 | 0.1 | 0.1 | 0.1 | -- | 0.1 |
| citric acid (50%) | ** | ** | ** | ** | ** | ** | ** | ** | ** | ** | ** |
| tetrasodium EDTA | 0.2 | 0.2 | 0.2 | 0.2 | 0.2 | 0.2 | 0.2 | 0.2 | 0.2 | 0.2 | 0.2 |
| benzophenone | 0.2 | 0.2 | 0.1 | 0.2 | 0.2 | 0.2 | 0.2 | 0.2 | 0.2 | 0.2 | 0.2 |
| fragrance | 0.22 | 0.2 | 0.2 | 0.2 | 0.22 | 0.22 | 0.22 | 0.22 | 0.22 | 0.22 | 0.22 |
| colorant | -- | 0.13 | 0.32 | 0.13 | 0.02 | -- | -- | -- | -- | -- | -- |
| cosmetic additive(s) | 0.25 | 0.2 | 0.25 | 0.1 | 0.45 | 0.45 | 0.45 | 0.25 | 0.25 | 0.2 | 0.2 |
| styrene/acrylates copolymer | -- | -- | -- | -- | -- | -- | -- | -- | -- | | 0.25 |
| preservative | 0.02 | 0.02 | 0.02 | 0.02 | 0.02 | 0.02 | 0.02 | 0.02 | 0.02 | 0.02 | 0.02 |
| d.i. water | q.s. | q.s. | q.s. | q.s. | q.s. | q.s. | q.s. | q.s. | q.s. | q.s. | q.s. |

| | | | | | | | | | | | |
|---|---|---|---|---|---|---|---|---|---|---|---|
| (** between 0.1 - 0.5%wt. of aqueous citric acid (50%wt. acid) was included in each of the example compositions in order to attain the pH indicated) | | | | | | | | | | | |

**Table 2**

| | Ex.1 | Ex.2 | Ex.3 | Ex.4 | Ex.5 | Ex.6 | Ex.7 | Ex.8 | Ex.9 | Ex.10 | Ex.11 |
|---|---|---|---|---|---|---|---|---|---|---|---|
| pH | 5.53 | 5.31 | 5.47 | 5- 5.5 | 4.83 | 5.28 | 5.32 | 3.85 | 4.08 | 5.73 | 5.49 |
| log₁₀ kill, *S.aureus* (ATCC 6538) | 5.53 | 5.16 | 5.16 | -- | 4.53 | 5.35 | 4.71 | 1.25 | 2.47 | 5.16 | 5.16 |
| log₁₀ kill, *Ecoli* (NCIB 10038) | 5.38 | 5.25 | 5.25 | -- | 5.28 | 5.28 | 4.31 | 5.27 | 5.13 | 5.28 | 5.28 |
| log₁₀ kill, *P.aeruginosa* (ATCC 15442) | 5.53 | 4.97 | 4.97 | -- | 5.21 | 2.57 | 1.09 | 5.28 | 5.28 | 5.20 | 5.20 |
| log₁₀ kill, *E.hirae* (ATCC 10541) | 5.18 | 5.1 | 5.1 | -- | 5.21 | 5.29 | 5.29 | 3.06 | 3.88 | 5.08 | -- |
| viscosity, cP(at 20°C) | 8000 | 6613 | 3413 | -- | 4267 | -- | -- | 9707 | 8640 | 3000 | -- |
| viscosity, cP (at 25°C) | 4587 | 4053 | 1707 | -- | -- | 2347 | 4480 | -- | -- | -- | 1600 |

**Table 3**

| | |
|---|---|
| polyethylene glycol distearate | HALLSTAR PEG 6000 DS (100%wt. actives) |
| lauramine oxide (30%) | AMMONYX LO (30%wt. actives) (ex. Stepan Inc.) |
| lauramide DEA | NINOL 55-LL (100%wt. actives) (ex. Stepan Inc.) |
| bis-[polyoxypropylene (2), polyoxyethylene (10) myristyl ether | CROMOLLIENT SCE, described to be a alkoxylated diester polymer; di-PPG-2 myreth-10 adipate (ex. Croda Inc.) |
| cetyl trimethyl ammonium chloride (30%) | AMMONYX CETAC-30 (30%wt. actives) (ex. Stepan Inc.) |
| benzalkonium chloride | BARQUAT MB50 (50%wt. actives) (ex. Lonza Inc.) |
| benzethonium chloride | LONZAGARD BTC (100%wt. actives) (ex. Lonza Inc.) |
| cocoamidopropyl betaine (30%) | VELVETEX BK-35 (30%wt.) (ex. Cognis) |
| glycerine | glycerine, laboratory grade (100%wt. actives) |
| propylene glycol | propylene glycol (100%wt.actives) (ex. Dow Chem. Co.) |
| citric acid (50%) | citric acid, aqueous dilution (50%wt. actives) |
| tetrasodium EDTA | TRILON B (powder) (100%wt. actives) (ex. BASF) |
| benzophenone | UVINUL MS 40 (ex. BASF) |
| fragrance | (proprietary composition) |
| colorant | (proprietary composition) |
| cosmetic additive | (proprietary composition(s) e.g., milk protein, aloe vera, etc.) |
| styrene/acrylates copolymer | opacifier, described as being a latex of a styrene/acrylate polymer (proprietary composition) provided as ACUSOL OP305 (ex. Rohm & Haas Co.) |
| preservative | KATHON CG (1-1.7%wt. actives) (ex. Rohm & Haas) |
| d.i. water | deionized water |

## Claims

1. A topical antimicrobial composition for application to the epidermis which comprises:
• one or more quaternary ammonium compounds exhibiting germicidal efficacy; and
• is free of anionic soaps and anionic surfactants;
wherein:
the composition exhibits at least 3 log kill efficacy at 60 seconds contact time of at least two of the microorganisms selected from the group consisting of: S.
*aureus, E. coli, P. aeruginosa* and *E.hirae*; and,
**characterised in that**
the composition further comprises a ternary thickening system comprising a benzophenone constituent, an oxyalkylenated constituent, and a fatty alkanolamide constituent.

2. A topical antimicrobial composition according to claim 1 which has a pH of between 4.0 to 7.0.

3. A topical antimicrobial composition according to either claim 1 or claim 2 which has a pH of 5.5 or less.

4. A topical antimicrobial composition according to claim 3 which has a pH of 5 - 5.5.

5. A topical antimicrobial composition according to any preceding claim which has a viscosity of 1500 - 20,000 cP at 25°C.

6. A topical antimicrobial composition according to any preceding claim which further comprises a nonionic surfactant selected from the group consisting of ethoxylated alkylphenols, ethoxylated and propoxylated fatty alcohols, polyethylene glycol ethers of methyl glucose, polyethylene glycol ethers of sorbitol, ethylene oxidepropylene oxide block copolymers, ethoxylated esters of fatty (C₆ -C₂₄) acids, condensation products of ethylene oxide with long chain amines or amides, amine oxide compounds and mixtures thereof.

7. A topical antimicrobial composition according to any preceding claim which further comprises one or more substances selected from the group consisting of hydrophilic or lipophilic gelling agents, hydrophilic or lipophilic active agents, further thickeners, humectants, opacifiers, preservatives, antioxidants, solvents especially organic solvents, pH adjusting agents, pH buffers, chelating agents, fragrances or other materials which provide an aromatherapy benefit, fillers, preservatives, dyestuffs or colorants, and light stabilizers including UV absorbers.

8. A topical antimicrobial composition according to any preceding claim which comprises:
o from 0.001 to 2.5 wt% of one or more quaternary ammonium compounds exhibiting germicidal efficacy;
o from 0.01 to 25 wt% of a ternary thickener system comprising a benzophenone constituent, an oxyalkylenated constituent, and a fatty alkanolamide constituent; and,
o from 0.01 to 7 wt% of a nonionic surfactant.

9. A topical antimicrobial composition according to claim 7 which comprises:
o from 0.01 to 1 wt% of one or more quaternary ammonium compounds exhibiting germicidal efficacy;
o from 0.1 to 10 wt% of a ternary thickener system comprising a benzophenone constituent, an oxyalkylenated constituent, and a fatty alkanolamide constituent;
o from 0.01 to 1 wt% of a nonionic surfactant;
o from 0.5 to 5 wt% of an organic solvent; and,
o an organic carboxylic acid.

## Patentansprüche

1. Topische antimikrobielle Zusammensetzung zur Applikation auf die Epidermis, die Folgendes umfasst:
• eine oder mehrere quartäre Ammoniumverbindungen mit keimtötender Wirkung; und
• frei von anionischen Seifen und anionischen Tensiden ist;
wobei:
die Zusammensetzung bei einer 60-sekündigen Kontaktzeit mit mindestens zwei der Mikroorganismen, ausgewählt aus der Gruppe bestehend aus: *S. aureus, E. coli, P. aeruginosa* und *E. hirae,* eine Abtötungswirksamkeit von mindestens 3 Log aufweist; und
**dadurch gekennzeichnet, dass**
die Zusammensetzung weiterhin ein ternäres Verdickungssystem, umfassend einen Benzophenon-Bestandteil, einen oxyalkylenierten Bestandteil und einen Fettalkanolamid-Bestandteil, umfasst.

2. Topische antimikrobielle Zusammensetzung nach Anspruch 1, die einen pH-Wert zwischen 4,0 bis 7,0 aufweist.

3. Topische antimikrobielle Zusammensetzung nach entweder Anspruch 1 oder Anspruch 2, die einen pH-Wert von 5,5 oder weniger aufweist.

4. Topische antimikrobielle Zusammensetzung nach Anspruch 3, die einen pH-Wert von 5-5,5 aufweist.

5. Topische antimikrobielle Zusammensetzung nach einem vorhergehenden Anspruch, die eine Viskosität von 1500 - 20 000 cP bei 25°C aufweist.

6. Topische antimikrobielle Zusammensetzung nach einem vorhergehenden Anspruch, die weiterhin ein nichtionisches Tensid, ausgewählt aus der Gruppe bestehend aus ethoxylierten Alkylphenolen, ethoxylierten und propoxylierten Fettalkoholen, Polyethylenglykolethern von Methylglucose, Polyethylenglykolethern von Sorbit, Ethylenoxid-Propylenoxid-Blockcopolymeren, ethoxylierten Estern von (C₆-C₂₄) -Fettsäuren, Kondensationsprodukten von Ethylenoxid mit langkettigen Aminen oder Amiden, Aminoxidverbindungen und Mischungen davon, umfasst.

7. Topische antimikrobielle Zusammensetzung nach einem vorhergehenden Anspruch, die weiterhin eine oder mehrere Substanzen, ausgewählt aus der Gruppe bestehend aus hydrophilen oder lipophilen Geliermitteln, hydrophilen oder lipophilen Wirkstoffen, weiteren Verdickungsmitteln, Feuchthaltemitteln, Opakisierungsmitteln, Konservierungsstoffen, Antioxidantien, Lösungsmitteln, insbesondere organischen Lösungsmitteln, Mitteln zum Einstellen des pH-Werts, pH-Puffern, Komplexbildnern, Duftstoffen oder anderen Substanzen, die einen Aromatherapie-Nutzen bereitstellen, Füllmitteln, Konservierungsstoffen, Farbstoffen oder Farbmitteln sowie Lichtstabilisatoren einschließlich UV-Absorbern, umfasst.

8. Topische antimikrobielle Zusammensetzung nach einem vorhergehenden Anspruch, die Folgendes umfasst:
o 0,001 bis 2,5 Gew.-% an einer oder mehreren quartären Ammoniumverbindungen mit keimtötender Wirkung;
o 0,01 bis 25 Gew.-% eines ternären Verdickungsmittelsystems, umfassend einen Benzophenon-Bestandteil, einen oxyalkylenierten Bestandteil und einen Fettalkanolamid-Bestandteil; und
o 0,01 bis 7 Gew.-% eines nichtionischen Tensids.

9. Topische antimikrobielle Zusammensetzung nach Anspruch 7, die Folgendes umfasst:
o 0,01 bis 1 Gew.-% an einer oder mehreren quartären Ammoniumverbindungen mit keimtötender Wirkung;
o 0,1 bis 10 Gew.-% eines ternären Verdickungsmittelsystems, umfassend einen Benzophenon-Bestandteil, einen oxyalkylenierten Bestandteil und einen Fettalkanolamid-Bestandteil; und
o 0,01 bis 1 Gew.-% eines nichtionischen Tensids;
o 0,5 bis 5 Gew.-% eines organischen Lösungsmittels; und
o eine organische Carbonsäure.

## Revendications

1. Composition antimicrobienne topique pour une application à l'épiderme, qui comprend :
- un ou plusieurs composés d'ammonium quaternaire présentant une efficacité germicide ; et
- est dépourvue de savons anioniques et d'agents tensioactifs anioniques ;
où
la composition présente au moins 3 log d'efficacité de destruction à un temps de contact de 60 secondes d'au moins deux parmi les microorganismes choisis dans le groupe constitué par *S. aureus, E. coli, P. aeruginosa* et *E. hirae ;* et
**caractérisée en ce que**
la composition comprend en outre un système épaississant ternaire comprenant un constituant de benzophénone, un constituant oxyalkyléné, et un constituant d'alcanolamide gras.

2. Composition antimicrobienne topique selon la revendication 1, possédant un pH de 4,0 à 7,0.

3. Composition antimicrobienne topique selon la revendication 1 ou bien la revendication 2, possédant un pH de 5,5 ou moins.

4. Composition antimicrobienne topique selon la revendication 3, possédant un pH de 5-5,5.

5. Composition antimicrobienne topique selon l'une quelconque des revendications précédentes, possédant une viscosité de 1500 - 20 000 cP à 25°C.

6. Composition antimicrobienne topique selon l'une quelconque des revendications précédentes, comprenant en outre un agent tensioactif non ionique choisi dans le groupe constitué par les alkylphénols éthoxylés, les alcools gras éthoxylés et propoxylés, les éthers de polyéthylène glycol et de méthylglucose, les éthers de polyéthylène glycol et de sorbitol, les copolymères blocs d'oxyde d'éthylène/oxyde de propylène, les esters éthoxylés d'acides gras en (C₆-C₂₄), les produits de condensation d'oxyde d'éthylène avec des amines ou des amides à chaîne longue, les composés d'oxyde d'amine, et des mélanges de ceux-ci.

7. Composition antimicrobienne topique selon l'une quelconque des revendications précédentes, comprenant en outre une ou plusieurs substances choisies dans le groupe constitué par les agents gélifiants hydrophiles ou lipophiles, les agents actifs hydrophiles ou lipophiles, d'autres épaississants, des humectants, des agent opacifiants, des conservateurs, des antioxydants, des solvants, notamment des solvants organiques, des agents d'ajustement du pH, des tampons de pH, des agents chélatants, des parfums ou autres matériaux fournissant un avantage en termes d'aromathérapie, des charges, des conservateurs, des teintures ou des colorants, et des photostabilisants y compris des agents anti-UV.

8. Composition antimicrobienne topique selon l'une quelconque des revendications précédentes, comprenant
- de 0,001 à 2,5% en poids d'un ou plusieurs composés d'ammonium quaternaire présentant une efficacité germicide ;
- de 0,01 à 25% en poids d'un système épaississant ternaire comprenant un constituant de benzophénone, un constituant oxyalkyléné, et un constituant d'alcanolamide gras ; et
- de 0,01 à 7% en poids d'un agent tensioactif non ionique.

9. Composition antimicrobienne topique selon la revendication 7, comprenant
- de 0,01 à 1% en poids d'un ou plusieurs composés d'ammonium quaternaire présentant une efficacité germicide ;
- de 0,1 à 10% en poids d'un système épaississant ternaire comprenant un constituant de benzophénone, un constituant oxyalkyléné, et un constituant d'alcanolamide gras ;
- de 0,01 à 1% en poids d'un agent tensioactif non ionique ;
- de 0,5 à 5% en poids d'un solvant organique ; et
- un acide carboxylique organique.
